# EUROPÄISCHE PATENTSCHRIFT

(11) **EP 0 515 439 B1**
(45) Veröffentlichungstag und Bekanntmachung des Hinweises auf die Patenterteilung: **08.06.1994**
(21) Anmeldenummer: 91903634.3
(22) Anmeldetag: 24.01.1991
(51) Int. Cl.: H01S 3/10, A61B 17/36

(54) **LASER MIT EINER EINSTELLEINRICHTUNG**
LASER WITH BEAM-INTENSITY ADJUSTMENT DEVICE
LASER AVEC DISPOSITIF DE REGLAGE

(30) Priorität: 15.02.1990 CH 489/90
(43) Veröffentlichungstag der Anmeldung: 02.12.1992
(73) Patentinhaber: NWL LASER-TECHNOLOGIE GMBH, D-91220 Schnaittach (DE)
(72) Erfinder: WEIMEL, Erich, D-8563 Schnaittach (DE)
(74) Vertreter: Keller, René, Dr.
(86) Internationale Anmeldenummer: EP9100137
(87) Internationale Veröffentlichungsnummer: WO9112640

(56) Entgegenhaltungen:
- DE-A- 3 339 370
- US-A- 4 692 924

## Beschreibung

Die Erfindung betrifft einen Laser gemäß dem Oberbegriff des Patentanspruchs 1.

Ein Laser dieser Art ist aus der DE-OS 33 39 370 bekannt. Der bekannte Laser sendet einen gepulsten, polarisierten Laserstrahl aus, dessen Intensität durch Verstellen eines Polarisationswürfels mit einem Betätigungsknopf verändert wird. Nach Loslassen des Betätigungknopfes wird ein Probeimpuls ausgelöst, die Energie dieses Impulses gemessen und gegebenenfalls nachgeregelt. Anstelle den Polarisationswürfel zu verstellen, kann auch die Spannung der Laser-Blitzlampe verändert werden.

Werden Laser in der Medizin oder bei heiklen Anwendungen der Materialbearbeitung eingesetzt, so muß deren Ausgangsleistung genau eingestellt und darf während des Betriebs nicht unwissentlich verändert werden, um Unfälle oder Verletzungen bzw. Ausschuß zu vermeiden.

Der Erfindung liegt die Aufgabe zugrunde, die Gefahr einer ungewollten Verstellung der Laserausgangsleistung zu vermeiden.

Die Lösung der Aufgabe ist Gegenstand des Patentanspruchs 1.

In den Patentansprüchen 2 bis 9 sind bevorzugte Ausführungsarten des erfindungsgemäßen Lasers beschrieben.

Im folgenden wird ein Beispiel des erfindungsgemäßen Lasers anhand eines schematischen Blockschaltbildes näher erläutert.

Der im Blockschaltbild dargestellte Nd:YAG-Festkörperlaser hat einen Laserresonator 1, eine Strahlführung 3 zur Führung und Fokussierung des Laserstrahls 5 sowie eine elektrische Steuereinrichtung 7.

Der Laserresonator 1 hat zwei Resonatorspiegel 9a und 9b, einen mit Neodym dotierten Kristall 11 aus Yttriumgranat als aktives Medium, eine Entladungslampe 13 zur Anregung des Kristalls 11 und einen Shutter 12 zum Ausschalten des Laserstahls 5.

Die Strahlführung 3 hat unmittelbar nach Austritt des Laserstrahls 5 aus dem Laserresonator 1 eine unter 45° stehende Glasplatte 15, welche als teildurchlässiger Spiegel wirkt und einen geringen Teil der Energie des Laserstrahls 5 auf eine Fotodiode 17 richtet. Die restliche, nahezu totale Energie des Laserstrahls 5 wird mit einer Optik 19 auf das zu bearbeitende Objekt 21 fokussiert.

Zwischen der Optik 19 und dem teildurchlässigen Spiegel 15 befindet sich eine massive, schwenkbare Klappe 23 als Ablenkelement, welche den Laserstahl 5 in ihrer Ablenklage, wie gestrichelt dargestellt, diffus in einen Hohlraum 25 als Laserstrahlabsorptionsvorrichtung reflektiert; in ihrer Ruhelage (ausgezogene Darstellung) befindet sich die Klappe 23 außerhalb des Laserstrahls 5. Zwischen der Klappe 23 und der Optik 19 ist ein sog. Sicherheitsverschluß 26 zum Abblocken des Laserstrahls 5 von der Optik 19 und damit vom zu bearbeitenden Objekt 21 vorhanden.

Die Fotodiode 17 ist über einen Verstärker 27 und eine Signalaufbereitungsschaltung 28 mit einem elektronischen Datenspeicher 29 verbunden. Der Speicher 29 ist mit einer Überwachungsschaltung 31, einer Anzeige 33 mit zwei Anzeigefeldern 32a und 32b sowie mit einer Speichersteuerung 35 und einem weiteren elektronischen Datenspeicher 37, welcher ebenfalls mit der Anzeige 33, der Überwachungssteuerung 31 und der Speichersteuerung 35 verbunden ist, verbunden. Die Signalaufbereitungsschaltung 28 digitalisiert das mit der Fotodiode 17 empfangene und mit dem Verstärker 27 verstärkte Signal entsprechend seiner Intensität und sendet es in einer kontinuierlichen Taktfolge zum Speicher 29, in dem jeweils der vorhergehende abgespeicherte Wert durch einen neuen der Signalaufbereitungsschaltung 28 überschrieben wird. Der jeweilige Wert in Speicher 29 wird auf dem Anzeigefeld 32a dargestellt.

Die Speichersteuerung 35 ist mit einer logischen Schaltung 39 verbunden, welche mit einer Betätigungsta- ste 41, einer Regelschaltung 43 und über eine Umschaltelektronik 46 mit einem Kontakt 44 einer Einstelleinrichtung 45 verbunden ist. Die Umschaltelektronik 46 ist mit einem Umschalttreiber 47 verbunden, welcher die Klappe 23 je nach Stellung des Kontaktes 44 in den Laserstrahl 5 hinein- (Ablenklage) bzw. aus diesem herausschwenkt (Ruhelage). Die Umschaltelektronik 46 ist mit der Regelschaltung 43 verbunden und bewirkt ebenfalls die Verstellung des Sicherheitsschalters 26, wobei dieser durch ein elektrisches Signal der Regelschaltung 43 in den Laserstrahl 5 hinein- oder herausgeschwenkt wird. Ein Lasernetzgerät 48 (Steuerorgan für die Entladungslampe 13) ist mit der Regelschaltung 43 verbunden und erhält von diesem Regelsignale zur Steuerung des Stroms durch die mit ihm verbundene Entladungslampe 13 im Laserresonator 1.

Die Einstelleinrichtung 45 hat ein Einstellelement 49, zwei im Blockschaltbild nur schematisch angedeutete Rasterscheiben 51a und 51b als Kupplungselemente, ein Drehpotentiometer 53 und den Kontakt 44. Das Einstellelement 49 ist ein auf einer Achse 55 befestigter, mit einem Anschlagrand 56 versehener Drehknopf 57, der mit einer Feder 59 soweit aus einer Frontplatte 61 einer nicht dargestellten Schalttafel herausgedrückt wird, bis sein Anschlagrand 56 an der Rückseite der Frontplatte 61 anliegt. Die Rasterscheibe 51a ist am zum Drehknopf 57 entgegengesetzten Ende der Achse 55 befestigt. Der Rasterscheibe 51a gegenüber liegt die Rasterscheibe 51b, welche über eine nicht dargestellte Achse auf das schematisch dargestellte Drehpotentiometer 53 wirkt.

Die Leistung des Laserstrahls 5 wird, wie oben dargestellt, durch den Strom, den sog. Pumplichtstrom, durch die Entladungslampe 13 verändert. Die Leistung des Laserstrahls 5 und der Strom durch die Entladungslampe 13 stehen in keinem linearen Verhältnis zueinander. Auch kann bei fest eingestelltem Pumplichtstrom die Laserausgangsleistung sich verändern, in der Regel abnehmen, sofern u. a. sich die Lichtemission der Entladungslampe 13 sowie die optische Güte des Laserresonators 1 ändert. Zur Einstellung der Laserleistung wird der Drehknopf 57 des Einstellelements 49 der Einstelleinrichtung 45 gegen die Kraft der Feder 59 in die Frontplatte 61 hineingedrückt. Hierdurch wird der Kontakt 44 geschlossen, die Regelschaltung 43 erhält über die Umschaltelektronik 46 und die logische Schaltung 39 ein elektrisches Signal, vorauf diese den Shutter 12 schließt, und sobald sie die Verschlußmeldung von Shutter 12 erhält, die Klappe 23 in die im Blockschaltbild gestrichelte Ablenklage einklappt. Befindet sich die Klappe 23 in ihrer eingeklappten Endposition, zieht die Regelschaltung 43 den Shutter 12 wieder aus dem Laserstrahl 5 heraus. Durch diesen Ablauf wird vermieden, daß an Kanten und Oberflächen gestreutes Laserlicht durch die Optik 19 austritt.

Ein Teil des aus dem Laserstrahl 5 mit dem teildurchlässigen Spiegel 15 ausgeblendeten Lichts gelangt in die Fotodiode 17. Der hierdurch erhaltene Fotostrom der Fotodiode 17 wird durch den Verstärker 27 verstärkt und mit der Signalaufbereitungsschaltung 28 über einen Zeitraum von einigen einhundert Millisekunden gemittelt, digitallsiert, und mit einer Taktfrequenz, welche dem reziproken Wert der Mittlungszeit entspricht, jeweils in den Speicher 29 eingelesen, wobei jeweils ein bereits in Speicher 29 stehender Wert überschrieben wird. Der Speicherwert wird sofort an das Anzeigefeld 32a weitergeleitet und dort angezeigt, wodurch im Anzeigefeld 32a immer der Momentanwert der Laserausgangsleistung abzulesen ist. Eine Mittelwertbildung wird vorgenommen, um kurzzeitige Schwankungen der Laserausgangsleistung durch eventuelles sog. Spiking oder andere kurzzeitige Intensitätsveränderungen eliminieren zu können.

Der Laserstrahl 5 fällt auf die Klappe 23 und wird durch diese diffus in den Hohlraum 25 reflektiert. Je nach der maximalen Leistung des Laserstrahls 5 sind die Außenwände des Hohlraums 25 mit Kühlrippen oder mit einer Wasserkühlung versehen, um die durch die Absorption des Strahls 5 anfallende Wärme abzuführen.

Ist der Drehknopf 57, wie oben beschrieben, eingedrückt, so greifen die beiden Rasterscheiben 51a und 51b ineinander, der Drehknopf 57 ist mechanisch mit dem Potentiometer 53 verbunden und der Widerstandswert des Potentiometer 53 kann durch Drehen des Drehknopfes 57 verändert werden. Im eingedrückten Zustand ist der Drehknopf 57 an das Potentiometer 53 gekoppelt. Die Veränderung des Widerstandswerts des Potentiometers 53 wirkt derart auf die Regelschaltung 43, daß ein hoher Widerstandswert einen hohen und ein tiefer einen niedrigen Strom durch die Entladungslampe 13 ergibt.

Bei einer Feineinstellung sollte vorteilhaft einige Sekunden gewartet werden bis sich ein thermisches Gleichgewicht im Laserresonator 1 eingestellt hat. Ist die gewünschte Laserleistung erreicht, so wird der Drehknopf 57 losgelassen, wobei er durch die Kraft der Feder 59 herausgedrückt wird, bis er mit seinem Anschlagrand 56 an der Rückseite der Frontplatte 61 anschlägt. Der Drehknopf 57 ist vom Potentiometer 53 entkoppelt. Zur Bestätigung des eingestellten Werts der Laserausgangsleistung muß innerhalb einer Sekunde die Bestätigungstaste 41 gedrückt werden, welche über die Schaltung 39 auf die Regelschaltung 43 und die Speichersteuerung 35 wirkt. In der Regelschaltung 43 wird hierdurch der eingestellte Strom durch eine interne Schaltung auf dem eingestellten Wert gehalten. Die Speichersteuerung 35 gibt an den Speicher 29 ein Signal, um den Wert der Laserausgangsleistung aus dem Speicher 29 in den Speicher 37 als Einstellwert zu übertragen und auf dem Anzeigefeld 32b darzustellen.

Für den Beginn der Laserbearbeitung wird eine mit der Regelschaltung 43 verbundene Auslösetaste 63 gedrückt. Hierdurch wird der Shutter 12 geschlossen, die Klappe 23 in ihre im Blockschaltbild ausgezogen dargestellte Ruhelage zurückgeklappt, der Verschluß 26 aus dem Laserstrahls 5 herausgezogen, und nachdem der Verschluß 26 seine Endposition außerhalb des Laserstrahls 5 erreicht hat, der Shutter 12 wieder geöffnet. Der Laserstrahl 5 wird nun durch die Optik 19 auf das Objekt 21 fokussiert und bearbeitet dieses, d. h. bohrt bei stillstehendem Objekt 21 ein Loch, schneidet, erwärmt, belichtet oder graviert bei bewegtem Objekt 21.

Während des Betriebs wird die Laserausgangsleistung über den teildurchlässigen Spiegel 15 und die Fotodiode 17 gemessen. Auf der Anzeige 33 ist dann, wie oben dargelegt, im Anzeigefeld 32a immer der Momentanwert der Laserausgangsleistung und im Anzeigefeld 32b der Einstellwert der Laserausgangsleistung sichtbar. Übersteigt die momentane Laserausgangsleistung den Einstellwert um mehr als 10%, so wird von der Überwachungsschaltung 31, welche beide Werte miteinander vergleicht über die Regelschaltung 43 und das Lasernetzgerät 48 der Strom durch die Entladungslampe 13 ausgeschaltet, um Ausschuß, Verletzungen, Unfall, etc. zu vermeiden.

Wird der Einstellwert der Laserausgangsleistung um 10% unterschritten, so ertönt ein Warnsignal, welches zur Nachregelung der Laserleistung auffordert. Bei einer größeren Abweichung wird der Strom abgeschaltet.

Anstelle den Drehknopf 57 eindrückbar zumachen, kann das Einstellelement 49 auch so konstruiert werden, daß der Drehknopf zur Veränderung der Laserausgangsleistung herausgezogen werden muß. Die beiden Rasterscheiben können dann beispielsweise als Zahnräder ausgebildet werden, welche nur bei herausgezogenem Drehknopf 57 ineinander greifen.

Anstelle den Drehknopf 57 herauszuziehen oder hereinzudrücken, kann er auch mit einem kapazitiven Sensor ausgerüstet werden, der jedesmal anspricht, wenn der Drehknopf mit der Hand angegriffen wird. In dem Augenblick, indem der Sensor anspricht, wird dann eine Verriegelung elektrisch aufgehoben und die Laserleistung kann verstellt werden. Wird dieser Drehknopf losgelassen, so wird er wieder elektrisch verriegelt. Die eingestellte Leistung kann, wie oben bereits beschrieben, ebenfalls über die Betätigungstaste 41 bestätigt werden.

Anstelle die Betätigungstaste 41 zur Bestätigung des eingestellten Laserleistungswertes nach mindestens einer Sekunde zu drücken, kann auch je nach Betriebsbedingungen ein anderer Zeitwert gewählt werden. Auch kann auf die Bestätigung ganz verzichtet werden, wenn keine Fehlbetätigungen des Drehknopfes 57 möglich sind.

Anstelle die Laserleistung durch Verändern des Stromes durch die Entladungslampe 13 zu verändern, kann die Laserleistung auch, sofern sie polarisiert ist, durch Verstellen eines Polarisationsteilers, der zwischen den Spiegel 9b und dem teildurchlässigen Spiegel 15 anzuordnen ist, verstellt werden. Anstelle eines Polarisationsteilers können auch bei unpolarisierter Laserausgangsleistung vier mit einer Antireflexschicht versehene zur Laserstrahlachse schwenkbare Glasplatten verwendet werden, wobei die Schwenkebene von je zwei Plattenpaaren senkrecht zueinander stehen und je zwei Platten eines Paares unter gleichem Winkel zur Strahlachse stehen, aber unter entgegengesetztem Schwenksinn geschwenkt werden, um eine räumliche Versetzung des Laserstrahls zu vermeiden. Die Glasplatten können am gleichen Ort wie der Polarisationsteiler eingesetzt werden.

Der Nd:YAG-Festkörperlasers kann ein kontinuierlicher Laser mit konstanter Ausgangsleistung oder mit einer Pulsfolge konstanter Frequenz oder Repetitionsfrequnez sein. Die konstante Frequenz bzw. Pulsfolge wird mit einem nicht dargestellten, aktiven Schaltelement, z. B. einem elektrooptischen Schalter, im Laserresonator 1 erzeugt. Je nach eingestellter Intensität der Entladungslampe 13 handelt es sich um eine Pulsfolge, welche der Frequenz des optischen Schalters oder ganzzahligen Teilen davon folgt. Je höher die Intensität der Entladungslampe desto kleiner ist der Teiler der Frequenz; es gibt auch Intensitätswerte, bei denen die Pulsfolge zwischen zwei und mehreren Frequenzwerten hin und herspringt. Da der Fotostrom der Fotodiode 17 von der Signalaufbereitungsschaltung 28 über einen Zeitraum von einigen einhundert Millisekunden gemittelt wird, kann auch die Steuereinrichtung 7 mit diesem Laser zusammen wirken, welcher ebenfalls als kontinuierliche Laser bezeichnet wird, sofern die Pulsfolgefrequenz größer 500 Hz ist. Anstelle eines kontinuierlichen Nd:YAG-Lasers lassen sich selbstverständlich auch andere kontinuierliche Laser, wie z. B. ein kontinuierlicher Argonlaser sowie andere Gas-, Ionen-, Excimerlaser, etc. verwenden.

Bei dem erfindungsgemäßen Laser kann die Ausgangsleistung nicht durch ungewolltes Berühren des Drehknopfes 57 verändert werden, da dieser zuerst hineingedrückt (oder herausgezogen) werden muß und erst dann die Einstellung erfolgen kann. Als weitere Sicherheit ist die Bestätigungstaste 41 vorgesehen, durch deren Betätigung erst ein mit dem Drehknopf 57 eingestellter neuer Leistungswert zur Veränderung der Ausgangsleistung übernommen wird.

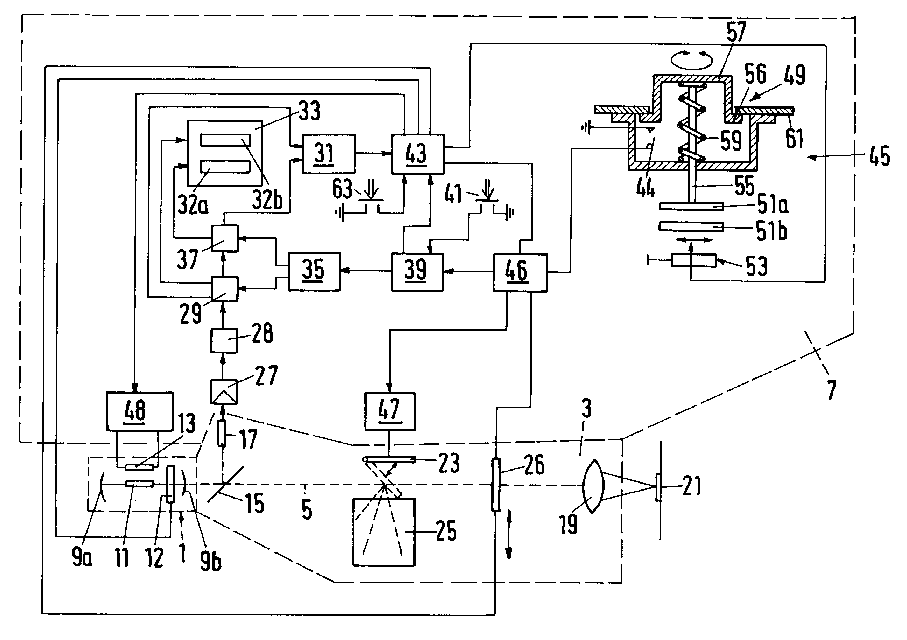

## Patentansprüche

1. Lasergerät mit einer ein manuell betätigbares Einstellelement (49) aufweisenden Einstelleinrichtung (45) zur Einstellung der Laserausgangsleistung und einem Anzeigegerät (33) für deren Anzeige, **gekennzeichnet durch** ein manuell einkuppelbares, mit dem Einstellelement zusammenwirkendes Kupplungselement (51a, 51b), mit dem die Einstellung des gewünschten Werts der Laserausgangsleistung nur im eingekuppelten Zustand möglich ist, und das beim Loslassen selbsttätig vom eingekuppelten in den ausgekuppelten Zustand übergeht, in dem ein Verstellen des Einstellelements (49) wirkungslos ist, damit eine Veränderung der Laserausgangsleistung durch ein ungewolltes Verstellen des Einstellelements (49) vermieden wird.

2. Lasergerät nach Anspruch 1, **gekennzeichnet durch** einen kontinuierlichen Laser zur Emission einer annähernd konstanten Ausgangsleistung oder einer kontinuierlichen Pulsfolgee von Pulsen mit einer Repetitionsfrequenz größer 500 Hz.

3. Lasergerät nach Anspruch 1 oder 2, **gekennzeichnet durch** eine Meßeinrichtung zur Bestimmung des Istwerts der Laserausgangsleistung mit einem Strahlteiler (15) zum Ausblenden eines Teils des Laserstrahls (5) und einem im ausgeblendeten Laserstrahlteil angeordneten lichtempfindlichen Element (17).

4. Lasergerät nach Anspruch 3, **gekennzeichnet durch** eine Überwachungsvorrichtung (31), welche den mit der Einstelleinrichtung (45) eingestellten Sollwert mit dem durch die Meßeinrichtung bestimmten Istwert der Laserausgangsleistung vergleicht und bei Überschreiten des Sollwerts um einen vorgegebenen Toleranzwert den Laserstrahl (5) abschaltet.

5. Lasergerät nach Anspruch 4, **dadurch gekennzeichnet**, daß die Überwachungsvorrichtung (31) den Laserstrahl (5) bei Unterschreiten des Sollwerts um einen zweiten Toleranzwert abschaltet.

6. Lasergerät nach einem der Ansprüche 3 bis 5, da**durch gekennzeichnet**, daß das Anzeigegerät (33) zur Anzeige sowohl des mit der Einstelleinrichtung eingestellten Sollwerts als auch des durch die Meßeinrichtung bestimmten Istwerts der Laserausgangsleistung ausgebildet ist.

7. Lasergerät nach einem der Ansprüche 1 bis 6, da**durch gekennzeichnet**, daß das Einstellelement (49) derart ausgebildet ist, daß das mit ihm zusammenwirkende Kupplungselement (51a, 51b) mit nur einer Hand einkuppelbar und der gewünschte Wert der Laserausgangsleistung im eingekuppelten Zustand mit nur einer Hand veränderbar ist.

8. Lasergerät nach einem der Ansprüche 1 bis 7, **gekennzeichnet durch** eine Laserstrahlabsorptionseinrichtung (25) und ein aus einer Ruhelage außerhalb des Laserstrahls (5) in eine Ablenklage zur Ablenkung des Laserstrahls (5) in die Laserstrahlabsorptionseinrichtung (25) bewegbares Ablenkelement (23), das beim Einkuppeln des Kupplungslements (51a, 51b) von der Ruhein die Ablenklage bewegt und beim Auskuppeln des Kupplungselements (51a, 51b) in die Ruhelage zurückbewegt wird.

9. Lasergerät nach einem der Ansprüche 1 bis 8, **gekennzeichnet durch** einen elektronischen Datenspeicher (37) und eine Bestätigungstaste (41), nach deren Betätigung innerhalb eines vorgegebenen Zeitintervalls, welches bei Erreichen des ausgekuppelten Zustands der Kupplungselemente (51a, 51b) beginnt, der mit dem Einstellelement (49) im eingekuppelten Zustand eingestellte Wert der Laserausgangsleistung in den Datenspeicher (37) übernommen bzw. bei Nichtbetätigen oder Betätigung außerhalb des Zeitintervalls nicht übernommen wird, sowie ein Steuerorgan (48), welches die Laserausgangsleistung beim Übergang des Kupplungslements (51a, 51b) vom eingekuppelten in den ausgekuppelten Zustand bzw. am Ende des vorbestimmten Zeitintervalls auf den in dem Datenspeicher (37) gespeicherten Wert einstellt.

## Claims

1. A laser appliance with an adjusting device (45), comprising a manually operable adjusting element (49), for adjusting the laser output, and with a display unit (33) for displaying the output, characterised by a manually couplable coupling element (51a, 51b) cooperating with the adjusting element, by means of which coupling element it is only possible to set the desired value of the laser output in the coupled state, and which when released automatically changes from the coupled to the uncoupled state in which setting the adjusting element (49) has no effect, so that a change in the laser output due to an undesired adjustment of the adjusting element (49) is prevented.

2. A laser appliance according to claim 1, characterised by a continuous laser for the emission of an approximately constant output or a continuous train of pulses with a repetition frequency greater than 500 Hz.

3. A laser appliance according to claim 1 or 2, characterised by a measuring device provided for determining the actual value of the laser output, with a beam splitter (15) for selecting a part of the laser beam (5) and a light-sensitive element (17) disposed in the selected part of the laser beam.

4. A laser appliance according to claim 3, characterised by a monitoring apparatus (31) which compares the set value set by means of the adjusting device (45) with the actual value of the laser output determined by the measuring device and which switches off the laser beam (5) when the set value is exceeded by a predetermined tolerance value.

5. A laser appliance according to claim 4, characterised in that when the output falls below the set value by a second tolerance value the monitoring apparatus (31) switches off the laser beam (5).

6. A laser appliance according to any one of claims 3 to 5, characterised in that the display unit (33) is designed for displaying both the set value set by means of the adjusting device and the actual value of the laser output determined by the measuring device.

7. A laser appliance according to any one of claims 1 to 6, characterised in that the adjusting element (49) is designed in such a way that the coupling element (51a, 51b) cooperating with it can be coupled using only one hand and the desired value of the laser output can be changed using only one hand in the coupled state.

8. A laser appliance according to any one of claims 1 to 7, characterised by a laser beam absorption device (25) and a deflection element (23) which can be moved from an inoperative position outside the laser beam (5) into a deflection position for deflecting the laser beam (5) into the laser beam absorption device (25), which deflection element is moved from the inoperative position into the deflection position on coupling the coupling element (51a, 51b) and is moved back into the inoperative position on uncoupling the coupling element (51a, 51b).

9. A laser appliance according to any one of claims 1 to 8, characterised by an electronic memory (37) and an operating key (41), after the operation of which within a predetermined time interval which begins on arriving at the uncoupled state of the coupling elements (51a, 51b) the value of the laser output set in the coupled state by means of the adjusting element (49) is transferred into the memory (37) or is not transferred in the event of non-operation or operation outside the time interval, and by a control element (48) which adjusts the laser output to the value stored in the memory (37) on the transition of the coupling element (51a, 51b) from the coupled to the uncoupled state or at the end of the predetermined time interval.

## Revendications

1. Appareil à laser, comprenant un dispositif de positionnement (45) comportant un élément de réglage (49) actionnable à la main, pour ajuster la puissance de sortie du laser et un appareil d'affichage (33) pour donner celle-ci, caractérisé par un élément d'accouplement (51a, 51b), qui peut être monté à la main et coopère avec l'élément de réglage, au moyen duquel l'ajustement de la valeur souhaitée de la puissance de sortie du laser n'est possible que dans l'état accouplé et qui passe automatiquement, lorsqu'il est relâché, de l'état accouplé à l'état désaccouplé où le déplacement de l'élément de réglage (49) est empêché afin que soit évitée une modification de la puissance de sortie du laser par un déplacement involontaire de cetélément de réglage (49).

2. Appareil à laser selon la revendication 1, caractérisé par un laser continu, de manière à émettre une puissance de sortie approximativement constante ou une séquence continue d'impulsions dans laquelle la fréquence de répétition de ces impulsions est supérieure à 500 Hz.

3. Appareil à laser selon la revendication 1 ou 2, caractérisé par un dispositif de mesure servant à déterminer la valeur réelle de la puissance de sortie du laser au moyen d'une lame séparatrice (15), servant à séparer une partie du faisceau laser (5), et d'un élément sensible à la lumière (17) , disposé dans la partie séparée du faisceau laser.

4. Appareil à laser selon la revendication 3, caractérisé par un dispositif de surveillance (31) qui compare la valeur de consigne de la puissance de sortie dulaser, ajustée par le dispositif de réglage (45), et la valeur réelle de cette puissance, déterminée par le dispositif de réglage, et qui interrompt l'émission du faisceau laser (5) lorsque la valeur de consigne est dépassée, en tenant compte d'une valeur de tolérance prédéterminée.

5. Appareil à laser selon la revendication 4, caractérisé en ce que le dispositif de surveillance (31) interrompt l'émission du faisceau laser (5) lorsque la valeur de consigne n'est pas atteinte, en tenant compte d'une deuxième valeur de tolérance.

6. Appareil à laser selon l'une des revendications 3 à 5, caractérisé en ce que l'appareil d'affichage (33) est réalisé de manière à donner aussi bien la valeur de consigne de la puissance de sortie du laser, réglée par le dispositif de réglage, que la valeur réelle de cette puissance, déterminée par le dispositif de mesure.

7. Appareil à laser selon l'une des revendications 1 à 6, caractérisé en ce que l'élément de réglage (49) est réalisé d'une manière telle que l'élément d'accouplement (51a, 51b), qui coopère avec lui, peut être accouplé d'une seule main et la valeur souhaitée de la puissance de sortie du laser peut être modifiée d'une seule main dans l'état accouplé.

8. Appareil à laser selon l'une des revendications 1 à 7, caractérisé par un dispositif d'absorption (25) du faisceau laser et par un moyen de déviation, qui peut être déplacé depuis une position de repos située en dehors du faisceau laser (5) vers une position de déviation servant à dévier le faisceau laser (5) dans le dispositif d'absorption (25), déplacé de la position de repos vers la position de déviation lorsque l'élément d'accouplement (51a, 51b) est accouplé et qui est ramené dans la position de repos lorsque l'élément (51a, 51b) est désaccouplé.

9. Appareil à laser selon l'une des revendications 1 à 8, caractérisé par une mémoire électronique (37) de données et par une touche d'actionnement (41) après la commande de laquelle, à l'intérieur d'un intervalle de temps prédéterminé qui commence lorsqu'est atteint l'état accouplé des éléments d'accouplement (51a, 51b), la valeur de la puissance de sortie dulaser, réglée dans l'état accouplé à l'aide de l'élément de couplage (49), est mémorisée dans la mémoire (37) de donnée ou n'est pas mémorisée si la touche n'est pas actionnée ou si l'actionnement s'effectue en dehors de l'intervalle de temps, ainsi que par un organe de commande (48) qui règle la puissance de sortie du laser à la valeur mémorisée dans la mémoire (37) de données lors du passage de l'élément d'accouplement (51a, 51b) depuis l'état accouplé vers l'état désaccouplé ou à la fin de l'intervalle de temps prédéterminé.
